# EUROPEAN PATENT SPECIFICATION

(11) **EP 2 363 080 B1**
(45) Date of publication and mention of the grant of the patent: **15.07.2015**
(21) Application number: 11165170.9
(22) Date of filing: 14.12.2005
(51) Int. Cl.: A61B 17/064, A61F 2/30

(54) **Bone anchor for intervertebral disc prosthesis**
Knochenanker für Bandscheibenprothese
Ancre osseux pour prothèse de disque intervertébral

(30) Priority: 22.12.2004 FR 0413728
(43) Date of publication of application: 07.09.2011
(62) Divisional of application: 05857774.3
(73) Proprietor: LDR Medical, 10430 Rosières près Troyes (FR)
(72) Inventor: Zeegers, Willem, 6418 PP Heerlen (NL)
(74) Representative: Debay, Yves

(56) References cited:
- WO-A1-01/70141
- WO-A2-03/005939
- DE-A1- 4 328 690
- FR-A1- 2 846 550
- US-A1- 2003 195 514

## Description

The present invention relates to an intervertebral disc prosthesis, intended to be substituted for fibro-cartilaginous discs ensuring a bond between the vertebrae of the spinal column.

Various types of intervertebral disc prostheses are known in the prior art. Numerous prostheses, such as for example in the patent application WO 02 089 701 and WO 2004/041129, are constituted in a lower plate and an upper plate forming a sort of cage around a central core. A part of these prostheses enables the upper plate to swivel in relation to the central core and optionally permits the central core to slide in relation to the lower plate. This sliding of the central core in relation to the lower plate is an essential characteristic, as it must allow spontaneous positioning of the core in the ideal position to absorb constraints imposed on the prosthesis, during movements made by the patient wearing the prosthesis. The displacement of the core, co-operating with at least a plate about an uneven surface, enables an inclination between the plates of the prosthesis which facilitates the mobility of the patient wearing the prosthesis. The displacement of the core also prevents it from creeping under load, when subjected to major constraints. A part of these prostheses have osseous anchorage means allowing to attach these prostheses to the vertebrae between which they are intended to be inserted.

However, the size of the vertebrae varies greatly from person to person, for a same vertebra in a given position in the spinal column, but also for a given person depending on the position of the vertebrae in the spinal column between which a prosthesis is intended to be inserted. The intervertebral disc prostheses must be of a suitable size for the vertebrae between which they are intended to be inserted, depending on the person and on the position of these vertebrae in the spinal column. Moreover, depending on the spinal column disorder of the patient wearing the prosthesis, it is sometimes preferable that the prosthesis allows a correction of this disorder. The prostheses can thus be used to correct an inclination defect of the vertebrae, such as, for example, lordosis. To have prostheses that are suitable for the as big a majority of cases as possible, a large number of prostheses with different plate sizes and inclinations must therefore be envisaged. This multiplicity of prostheses has the major inconvenience of high manufacturing costs and high stock levels. In this context, it is beneficial to propose a prosthesis that has a structure allowing it to be adapted to different sizes of vertebrae and allowing for different inclinations of the plates. Such a prosthesis would reduce stock levels and manufacturing costs.

Document DE 43 28 690 A1 teaches an implant that is anchored by anchor called staple which is manufactured from a bent, broad band. The staple possesses two legs linked together by curved part allowing the staple to interlock onto a bridge of the implant. The staple also possesses a constriction allowing the staple to maintain the bridge of the implant in the curved part of the staple by clasping. Such staple is especially suitable for open implants which have a bridge that links two parts of an implant or which may be provided with a separate bridge for the staple. This type of implant does not possess plates forming a sort of cage around a central core thus it does not give a solution for prosthesis that includes plates. Moreover, the leg shape of the staple is not efficient for the fixing of the implant onto the bone.

The aim of the present invention is to propose an intervertebral disc prosthesis allowing limited movements of the different pieces of the prosthesis between one another and comprising a core used to restrict its displacement in at least one direction. A same prosthesis can be adapted to different sizes of vertebrae at reduced costs.

The invention of this divisional application is defined in claim1. Further embodiments are defined in the dependent claims.

Other features and advantages of the invention will emerge more clearly from the description herein below, given in reference to the attached diagrams, in which:
- figure 1 illustrates an exploded perspective view of the different elements of the prosthesis as described herein
- figure 2 illustrates an exploded perspective view of the different elements of the prosthesis
- figure 3 illustrates a perspective view of the prosthesis
- figures 4A and 4B respectively illustrate a bottom view and a cross section view along plane A-A in figure 4A, of the upper plate equipped with its anatomic adaptation element, figures 4C and 4D respectively illustrate a plan view and a cross section view along plane B-B in figure 4C, of the upper plate equipped with its anatomic adaptation element,
- figure 5A illustrates a bottom view of the upper plate equipped with its anatomic adaptation element, and figures 5B and 5C illustrate cross section views respectively along plane C-C and plane D-D in figure 5A, of the upper plate equipped with its anatomic adaptation element,
- figures 6A and 6B illustrate bottom views of a part of the upper plate equipped with its anatomic adaptation element,
- figures 7A and 7B illustrate perspective views of the lower plate equipped with its anatomic adaptation element,
- figures 8A and 8B respectively illustrate a bottom view and a cross section view along plane E-E in figure 8A, of a part of the lower plate equipped with its anatomic adaptation element whose fixation means are open, figures 8C and 8D respectively illustrate a bottom view and a cross section view along plane F-F in figure 8C, of the same embodiment as in figures 8A and 8B, but with the fixation means of the anatomic adaptation element closed and locked,
- figures 9A and 9B respectively illustrate a bottom view and a cross section view along plane G-G in figure 9A, of a part of the lower plate equipped with its anatomic adaptation element whose fixation means are open, figures 9C and 9D respectively illustrate a bottom view and a cross section view along plane H-H in figure 9C, of the same embodiment as in figures 9A and 9B, but with the fixation means of the anatomic adaptation element closed and locked,
- figures 10A and 10B illustrate perspective views of, respectively, the prosthesis comprising osseous anchorage means and one of the osseous anchorage means
- figures 11A and 11B respectively illustrate a perspective view of the prosthesis comprising osseous anchorage means and a cross section view along plane I-I of figure 11A.

The intervertebral disc prosthesis described herein is constituted in an upper plate (1) articulated in relation to a lower plate (2) by means of a core (3) and each of the plates (1, 2) is equipped with an anatomic adaptation element (11, 22) allowing to adjust the overall size of the prosthesis to the size of the vertebrae between which the prosthesis is intended to be inserted. Thus, thanks to the anatomic adaptation elements (11, 22), a single unit constitutes two plates (1, 2) and the core (3) can be used for different sizes of vertebrae, which has the advantage of substantially reducing the cost of manufacturing prostheses and their variances. The advantage of the prosthesis is that it comprises simple parts whose anatomic adaptation elements (11, 22) can be sized so as to adapt to different vertebrae of the spinal column, for example, the adjust the thickness of the prosthesis to the intervertebral gap and/or adjust the inclination of the plates (1, 2) of the prosthesis to the inclination of the vertebrae of the patient. Even though the anatomic adaptation elements (11, 22) allow themselves to adjust the prosthesis to different sizes of vertebrae , the plates (1, 2) and the core (3) of differing sizes and shapes can naturally be used if needs be.

The two anatomic adaptation elements (11, 22) of the prosthesis consist in an upper element (11) and a lower element (12). The upper element (11) has, on one hand, an upper surface (110) of which at least a part presents a surface in contact with a lower surface of a first vertebra and has, on the other hand, a lower surface (111) of which at least a part presents a surface in contact with a part of the upper plate (1). The lower element (22) has, on one hand, a lower surface (220) of which at least a part presents a surface in contact with an upper surface of a second vertebra and has, on the other hand, an upper surface (222) of which at least a part presents a surface in contact with a part of the lower plate (2). Each of the two anatomic adaptation elements (11, 22) is fixed onto the plates (1, 2) via respective fixation means (113, 223).

The core (3) is of slight thickness (from 3 to 15 mm, depending on the vertebrae between which the prosthesis is to be inserted). For good absorption of the constraints, the core (3) could, for example, be made of polyethylene, a compressible material simulating the physical properties of elasticity of natural intervertebral discs.

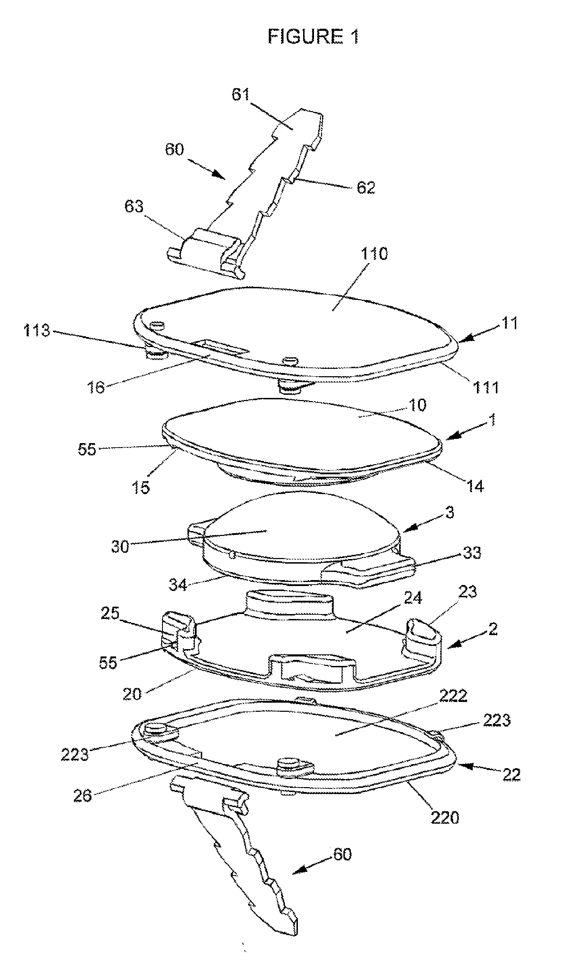

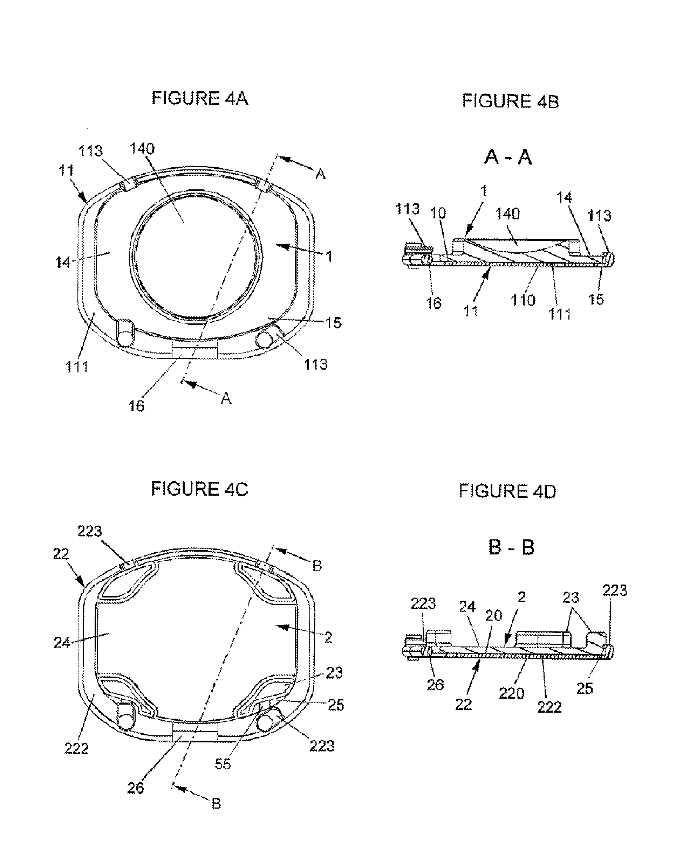

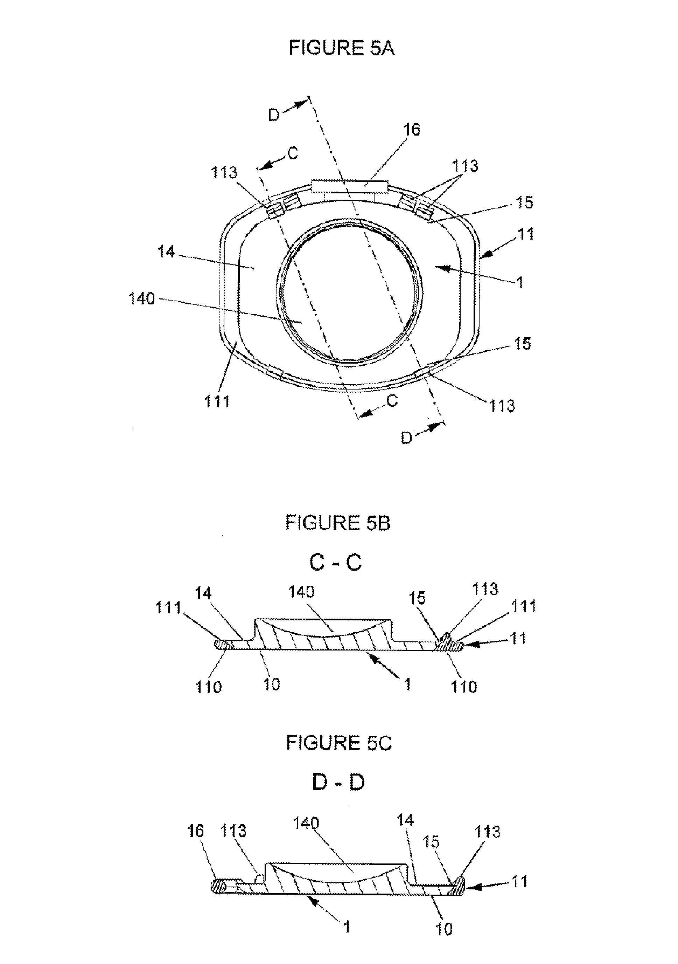

The core (3) has a convex part on at least a part of at least one of its upper (30) and lower (34) surfaces. In the embodiments illustrated in figures 1 to 9, it is the upper surface (30) of the core (3) which is convex and complementary with a concave part (140) of the lower surface (14) of the upper plate (1), whereas the lower surface (34) of the core (3) is plane and complementary with at least a plane part of the upper surface (24) of the lower plate (2). The concave part (140) of the lower surface (14) of the upper plate (1), as particularly visible in figures 4A, 4B, 5A, 5B and 5C, has a circular periphery. In other possible embodiments (not shown), it is a part of the lower surface (34) of the core (3) that can be convex and complementary with a concave part of the upper surface (24) of the lower plate (2), whereas the upper surface (30) of the core (3) is plane and complementary with at least a plane part of the lower surface (14) of the upper plate (1). In other embodiments (not shown), the concave surface lies on a part of one of the upper (30) and lower (34) surfaces of the core (3) and co-operates with a convex surface which lies on a part of a surface of one of the plates (1, 2). In these possible different embodiments (not shown) of the invention, the non convex or non concave surface of the core (3) can respectively be concave or convex, for example very slightly.

In the embodiments illustrated in figures 1 to 9, the concave part (140) of the lower surface (14) of the upper plate (1) complementary with the convex part of the upper surface (34) of the core (3) allows to incline the upper plate (1) when the patient wearing the prosthesis bends over. The cooperation between the concave surface (140) and the convex surface (34) presents a surface of articulation with the prosthesis, thanks to this inclination of the upper plate (1) in relation to the core (3). The centre of this articulation is naturally at the tip of the convex surface (34) of the core (3). In the illustrated embodiments, the lower surface of the core (3) and the upper surface of the lower plate (2) are plane so as to permit clearance of the core (3) in relation to the lower plate (2), both in translation according to an axis substantially parallel to the lower plate (2), and in rotation about an axis substantially perpendicular to the lower plate (2). During movements by the patient wearing the prosthesis, this inclination of the upper plate (1) and this clearance of the core will allow displacement of the core (3) towards the ideal position to absorb the constraints applied to the prosthesis. The movement between the upper plate (1) and the core (3), as well as the clearance of the core (3) in relation to the lower plate (2) thus allow the patient to move, and, optionally, to eliminate the defects of positioning the prosthesis. This clearance likewise has the advantage of preventing premature wear due to the constraints applied to the prosthesis.

Irrespective of the embodiment, the core (3) also has male or female co-operation means (33) complementary with respectively female or male cooperation means (23) present on at least one of the plates (1, 2). These male and female co-operation means (23, 33) situated in the vicinity of the edges of at least one plate (1, 2) and of the core (3) limit, without excessive friction, the movements in translation of the core (3) in relation to this plate (1, 2), according to an axis substantially parallel to this plate (1, 2), and limiting or suppressing the movements in rotation of the core (3) in relation to this plate (1, 2), about an axis substantially perpendicular to this plate (1, 2). The dimensions of each male co-operation means (33) can be slightly less than those of each female co-operation means (23) so as to allow slight clearance between the core (3) and the plate (1, 2) equipped with these co-operation means. On the contrary, the dimensions of each male cooperation means (33) can also be substantially the same as those of each female co-operation means (23) so as to prevent any clearance between the core (3) and the plate (1, 2) equipped with these co-operation means.

In the embodiment in figures 1 to 3, the core (3) has male co-operation means (33) complementary with female co-operation means (23) present on the lower plate (2). The male co-operation means (33) of the core (3) are, for example, hasps or blocks substantially parallelepiped in shape, present on the side edges of the core (3), as particularly visible in figures 1 to 3. The female co-operation means (23) can consist, for example, in four walls situated, in pairs, on each of the two side edges of the lower plate (2). These walls could be curved to the centre of the prosthesis, so as to cover at least a part of the male co-operation means (33) of the core (3) and avoid lifting the core (3) and the upper plate (1). These co-operation means (23, 33) also prevent the core (3) from ejecting out of the prosthesis, in the event of too much constraint on the prosthesis. In an alternative embodiment (not shown), the dimensions of each male co-operation means (33) of the core (3) are substantially the same as those of each female co-operation means (23) of the lower plate (2), so as to avoid any clearance of the core (3) in relation to the lower plate (2), both in translation and in rotation. In the latter case, the only permitted movement of the prosthesis is the inclining of the upper plate (1) in relation to the core (3). In an alternative embodiment (not shown), the core (3) has female co-operation means, consisting, for example, in complementary recesses of the male means present on the lower plate (2). These male means of the lower plate (2) can consist, for example, in two blocks or two nibs, for example curved to the interior of the prosthesis and facing one another on two edges of the lower plate (2). The nibs can, for example, be replaced by a block with a bore on which is fixed a hasp by way of a pin penetrating the bore. In another alternative embodiment (not shown), the lower plate (2) has half dog points. The core (3), by way of complement, has wells under its lower surface. The dimensions of the half dog points of the lower plate (2) and of the wells of the core (3) will be adapted, by choice, by a slight clearance of the core (3) in translation and in rotation or by no clearance, according to the desired result. In other alternatives (not shown), the co-operation means can be located on the core (3) and on the upper plate (1), instead of the lower plate (2).

The description of a first embodiment will now be considered in reference to figure 1. In this embodiment, the upper (11) and lower (12) anatomic adaptation elements consist in plates, called anatomic, which respectively cover the upper (1) and lower (2) plates. The upper (222) and lower (111) surfaces of respectively the lower (22) and upper (11) anatomic adaptation elements can have a reinforcement in which respectively the lower (2) and upper (1) plates are housed. In another alternative, these upper (222) and lower (111) surfaces of the anatomic adaptation elements can be plane and comprise stoppers which, as for the aforementioned reinforcements, prevent respectively the lower (2) and upper (1) plates from moving in relation to the anatomic adaptation elements. The upper (222) and lower (111) surfaces of respectively the lower (22) and upper (11) anatomic adaptation elements prolong respectively the upper (10) and lower (20) surfaces of the upper (1) and lower (2) plates, to present contact surfaces of the prosthesis with the adjacent vertebrae which are bigger than when there are no anatomic adaptation elements (11, 22). Different sizes of the anatomic plates of the anatomic adaptation elements (11, 22) can be adapted to a single unit created by the two plates (1, 2) and the core (3), to provide good contact between the prosthesis and the vertebrae of differing sizes.

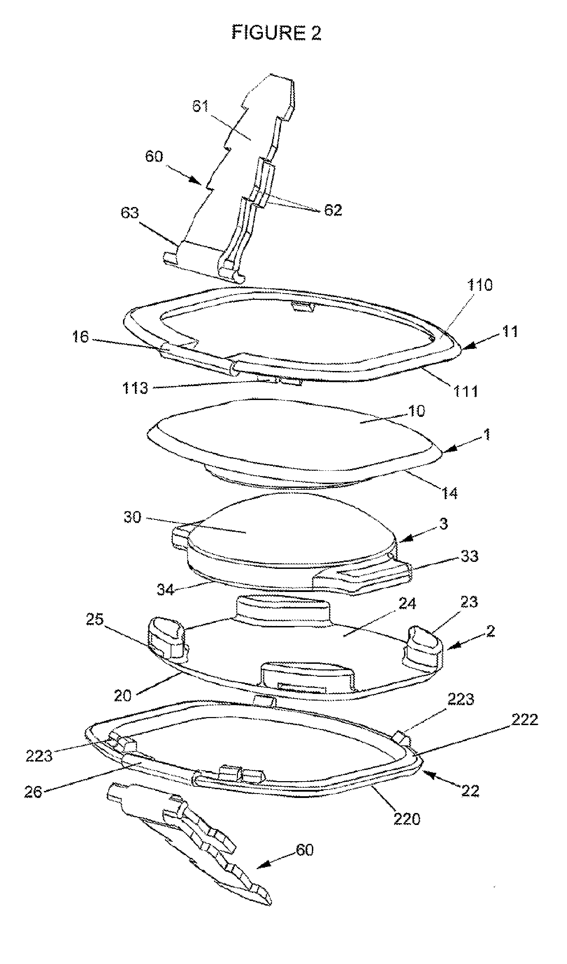

In the embodiment of the prosthesis illustrated in figure 2, the anatomic adaptation elements (11, 22) consist in crowns which surround the upper (1) and lower (2) plates. In this embodiment, the edges of the upper (10) and lower (20) surfaces of respectively the upper (1) and lower (2) plates are bevelled and complementary with respectively the lower (111) and upper (222) inside edges of respectively the upper (11) and lower (22) crowns. This inclined shape of the edges of the plates (1, 2) and of the anatomic adaptation crowns (11, 22) co-operates with the fixation means (113, 223) of the anatomic adaptation elements in order to maintain the anatomic adaptation crowns (11, 22) fixed in plane of respectively the upper (1) and lower (2) plates of the prosthesis. The anatomic adaptation crowns (11, 22) prolong respectively the upper (10) and lower (20) surfaces of respectively the upper (1) and lower (2) plates, to present contact surfaces of the prosthesis with the adjacent vertebrae which are bigger than when there are no anatomic adaptation elements (11, 22). In the same manner as for the aforementioned anatomic plates (11, 22), a single unit created by the two plates (1, 2) and the core (3) can thus be adjusted to vertebrae of differing sizes, thanks to different sizes of crowns of the anatomic adaptation elements (11, 22).

In all the embodiments the anatomic adaptation elements (11, 22) can symmetrically or asymmetrically prolong the upper (10) and lower (20) surfaces of respectively the upper (1) and lower (2) plates. Thus, for example, the anterior edge of the anatomic adaptation elements (11, 22) can have a bigger contact surface with the vertebrae than its posterior edge, so that the centre of articulation of the prosthesis (described above) is centred in relation to the natural axis of the spinal column, that meaning off centre to the rear of the vertebrae of a 2/3-1/3 section.

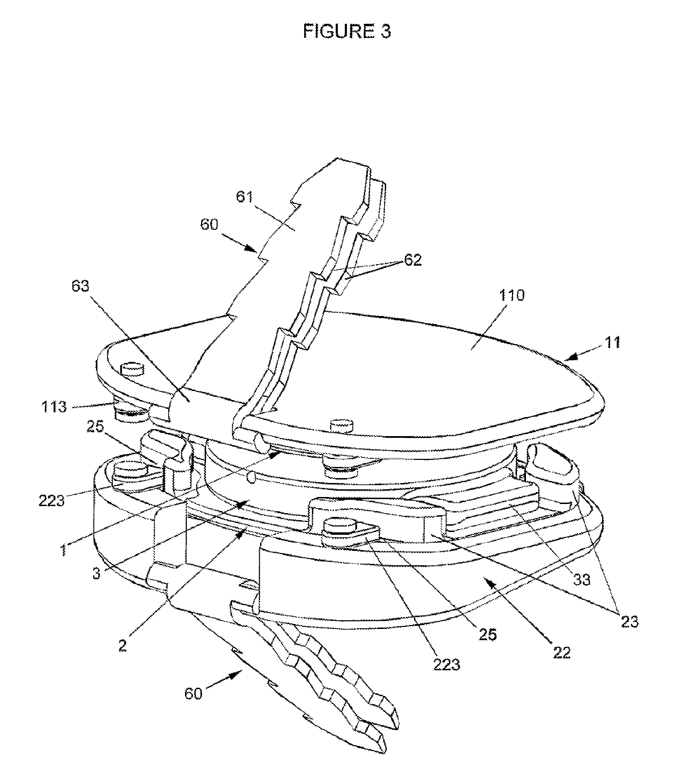

According to the chosen embodiments, the intervertebral disc prosthesis according to the invention allows, for example, to correct the defects of lordosis. The presence of an angle between the upper and lower surfaces of the prosthesis, in contact with the adjacent vertebrae, could be desirable. Such an angle could be obtained by making an upper plate (1), whose median planes representing its lower (14) and upper (10) surfaces create an angle. Another possibility consists in that it is the lower plate (2) whose median planes representing its lower (20) and upper (24) surfaces create an angle. Another possibility consists in that it is at least one of the anatomic adaptation elements (11, 22) whose median planes representing its lower and upper surfaces create an angle. Thus, a single unit made of the two plates (1, 2) and the core (3) can be used, for example, to induce or not lordosis, depending on which anatomic adaptation elements (11, 22) are associated to it. In the embodiment illustrated in figure 3, the lower surface (220) of the lower anatomic plate (22) creates an angle with its upper surface (222). Another possibility to obtain such an angle consists in a slightly offset position of the core (3) in relation to the centre of the prosthesis. This slightly offset position of the core (3) can, for example, be maintained thanks to an adjustable positioning of the male and female co-operation means (23, 33) between themselves. If the surgeon wishes, for example, that the prosthesis induces lordosis which remains within a range of values, he will select a prosthesis whose core (3) can have slight clearance in translation and in rotation in relation to the lower plate (2), but about a position imposing a slight permanent inclination of at least one of the plates, thanks to an accurate adjustment of the co-operation means (23, 33) between the core (3) and the lower plate (2). Thus, according to the chosen embodiment, the median planes representing the upper (110, 222) and lower (111, 220) surfaces of each of the anatomic adaptation elements (11, 22) can be substantially parallel or form an acute angle. The inclination obtained by such an angle allows to adapt the overall shape of the prosthesis to the anatomy of the spinal column or to possibly correct inclination defects of the vertebrae of the patient for whom the prosthesis is intended. The same anatomic adaptation elements (11, 22) can be assembled with different plates (1, 2) whose upper (10, 24) and lower (14, 20) surfaces create different angles. On the contrary, the plates (1, 2), whose upper (10, 24) and lower (14, 20) surfaces are parallel, are assembled with the anatomic adaptation elements (11, 22) whose upper (110, 222) and lower (111, 220) surfaces create different angles. This angle between the upper (10) surface of the upper plate (1) and the lower surface (20) of the lower plate (2) can be imposed either by the fact that the median planes representing the lower (20, 14) and upper (24, 10) surfaces of the lower plate (2) and/or the upper plate (1) create an angle, or by restricting, thanks to the co-operation means (23, 33), movements of the core (3) about a position imposing an inclination of at least one of the plates (1, 2).

Illustrated in figures 1 to 3 are the movable osseous anchorage means (60) according to the invention of the anatomic adaptation elements (11, 22). Advantageously, these osseous anchorage means (60) can be fixed onto the anatomic adaptation elements (11, 22) upon fixing them onto the plates (1, 2) and most of all upon inserting the prosthesis between the vertebrae. This feature allows the surgeon to easily position the prosthesis between the vertebrae and then insert the osseous anchorage means (60) once the prosthesis has been correctly positioned. In the embodiment presented in figure 1, these movable osseous anchorage means (60) consist in a plate (61) equipped with notches (62) oriented to resist against the removal of the plate (61) once it has been inserted into the vertebra. This plate (61) can, of course, be with or without notches (62) to resist against its removal from the vertebra. A far end of the plate (61) bears a part (63) curved to fold over itself. This curved part forms a kind of a hook intended to be interlocked onto an edge (16, 26) of an opening made in the vicinity of the periphery of the anatomic adaptation elements (11, 22). This edge (16, 26) of the opening creates a sort of rod onto which the osseous anchorage means (60) interlock. Indeed, the curved part (63) allows clasping the osseous anchorage means (60) onto the rod (16, 26) of the anatomic adaptation elements (11, 22). This rod can, of course, be replaced by any equivalent means allowing to clasp the osseous anchorage means (60). In the embodiments illustrated in figures 1 to 9, the rod (16, 26) is located on the anterior edge of the anatomic adaptation elements (11, 22) so as to allow the surgeon access once the prosthesis has been inserted between the vertebrae via anterior means (through accessing the vertebrae from their anterior face). If the implanting of the prosthesis is to be done via posterior means, the anatomic adaptation elements (11, 22) could have a rod (16, 26) located on the posterior edge. If the implanting of the prosthesis is to be done via lateral means, the anatomic adaptation elements (11, 22) could have a rod (16, 26) located on at least one of their edges. In the embodiment illustrated in figures 2 and 3, the hooked part (63), curved to fold over itself, of the notched plate (61) of the movable osseous anchorage means (60) of the anatomic adaptation elements (11, 22) prolong with a second plate (61) also equipped with notches (62) oriented to resist against the removal of the plate (61) once it has been inserted into the vertebra. In the embodiment illustrated in figure 2, this second plate (61) is shorter than the first plate and in the embodiment illustrated in figure 3, it is as long as the first plate. The fact that the osseous anchorage means (60) locks onto the rod (16, 26) allows it to have a variable angle which facilitates the attaching of the prosthesis. Indeed, depending on its encumbrance, the surgeon will have a choice of angles according to which he wishes to drive the osseous anchorage means (60) into the vertebrae. Moreover, the fact that the osseous anchorage means (60) can be inserted after positioning the prosthesis between the vertebrae this allows to adjust the relative position of the different elements (1, 2, 3) of the prosthesis. Indeed, the inserting of the prosthesis generates constraints on the elements of the prosthesis which are movable in relation to each other and they risk being badly position together. The surgeon can then, thanks to the invention, adjust the position of the prosthesis between the vertebrae and adjust the relative position of the elements of the prosthesis between themselves prior to definitively attaching the prosthesis.

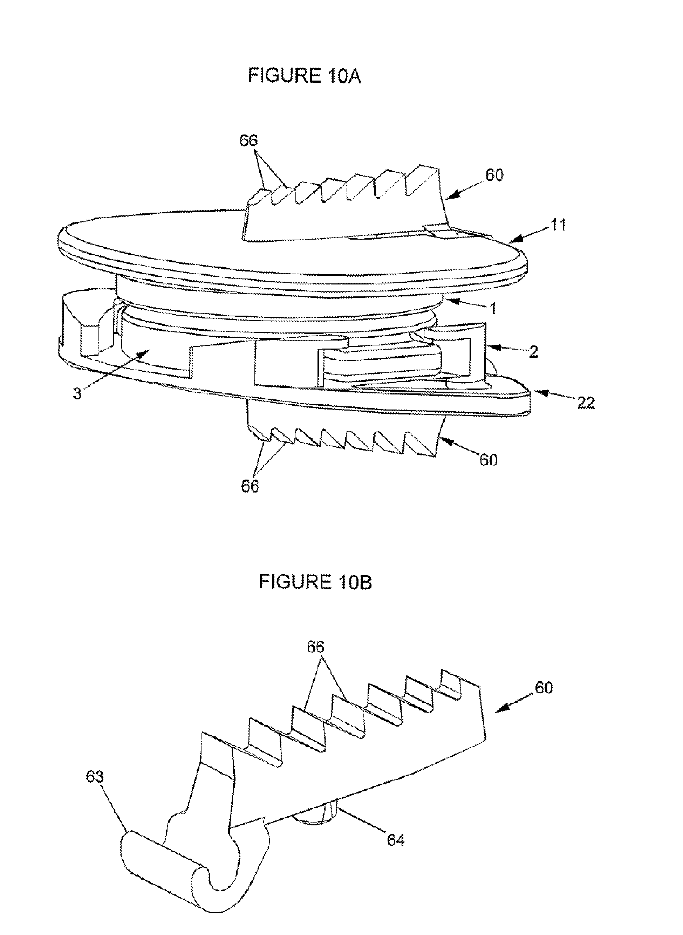

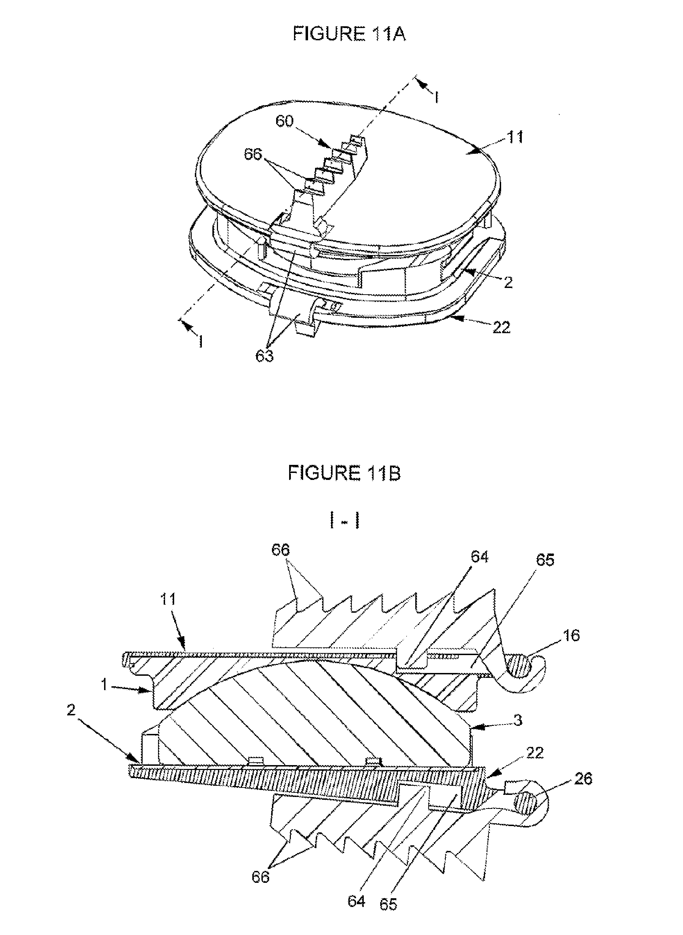

It is obvious that the above described prosthesis can comprise other osseous anchorage means (60) than those of the present invention. To give non-limitative examples, such osseous anchorage means (60) can consist of winglets fixed on the prosthesis as in the Patent Application WO03/039400 or of a stud nailed in the vertebra through the anatomic adaptation elements as in the Patent Application WO04/ 041129. One embodiment of the anchorage means (60) is presented on the figures 10A, 10B, 11A and 11 B. The osseous anchorage means (60) according to this embodiment consist of winglets comprising a hooked part (63), curved to fold over itself, so that the winglets can be adapted onto the anatomic adaptation elements. The hooked part (63) of the winglet, particularly visible on figure 10B, allows the anchorage means (60) to be interlocked onto the edge (16, 26) of an opening made in the vicinity of the periphery of the anatomic adaptation elements (11, 22), as particularly visible on figures 11A and 11B. This edge (16, 26) of the opening creates a sort of rod onto which the osseous anchorage means (60) interlock, as described above. The winglet further comprises a pin (64) (or a dowel) adapted to be inserted into a groove (65) present on the surface of the plate and/or the anatomic adaptation element on which the winglet is to be fixed, as particularly visible on figure 11B. The groove (65) and the pin (64) have dimensions adapted so that the pin (64) is secured into groove (65). For example, the pin (64) may have a substantially conical shape, with the larger diameter of the cone being at the base of the pin and the smaller diameter being at its end. The groove (65) may have its side walls adapted to cooperate with the conical shape of the pin (64) so that the pin tightly fits inside the groove and thus secures the osseous anchoring means (60) onto the anatomic adaptation elements (11, 22). For example, the width of the groove (65) may be larger at its surface than at its bottom. The osseous anchorage means (60) are thus fixed onto the prosthesis of the present invention by first interlocking the hooked part (63) onto the rod (16, 26) of the anatomic adaptation elements (11, 22) and by rotating the osseous anchorage means (60) around the rod until the pin (64) penetrates tightly into the groove (65) of the anatomic adaptation element (11, 22) and/or of the plate (1, 2). The winglet (60) can have a standard size for all the prosthesis made according to the present invention and the position of the pin (64) of the winglet (60) inside the groove (65) will vary as a function of the size of the anatomic adaptation elements (11, 22). Depending on the thickness of the anatomic adaptation elements (11, 22), the pin (64) penetrates into the anatomic adaptation element (11, 22) only or may traverse the anatomic adaptation element (11, 22) and penetrate into a groove (65) in the plates (1, 2), as shown for example on figure 11B for the upper plate (1). Since the anatomic adaptation element (11, 22) vary in size (diameter), their groove may have variable lengths and can be replaced by a hole having a variable distance from the rod (16, 26) so that the hole is adapted to receive the pin (64), but when the pin is designed to penetrate the plates also, the plates will have to include a groove because the distance of the pin from the periphery of the plates will vary depending on the size of the anatomic adaptation element (11, 22). Once secured onto the anatomic adaptation elements (11, 22), the winglets (60) are adapted to cooperate with a groove drilled in the surfaces of the adjacent vertebrae with which they are in contact. Thus, the surgeon may perform a groove in the surfaces of vertebrae between which the prosthesis is intended to be inserted. This groove in the vertebrae will naturally have an orientation relative to the sagittal plane that will depend on the position and orientation of the winglet. This orientation will be predetermined and will set and secure the orientation of the prosthesis. Similarly, the depth of the groove in the vertebrae and its extend from the periphery will be predetermined as a function of the size of the winglet (60) and will allow the surgeon to adjust the relative position of the various elements of the prosthesis and to predict the position of the prosthesis relative to the natural axis of the vertebrae. The winglets comprise notches (66) on their surfaces which are intended to be in contact with the bottom of the groove performed in the vertebrae. These notches (66) of the winglets (60) will resist against the ejection of the prosthesis from inside its housing between the vertebrae, for example when strong constraints are applied to the prosthesis. It is obvious (particularly from the figure 11B showing both embodiments) that the hooked part (63) of the winglets (60) can be oriented so that they are to be interlocked onto the rod (16, 26) by being inserted inside the opening made in the vicinity of the periphery of the anatomic adaptation elements (11, 22) or by being inserted from outside this opening.

It is obvious that the osseous anchoring means (60) described above are particularly adapted to the anatomic adaptation elements (11, 22) but may also be adapted to the plates of other types of intervertebral disc prosthesis having plates comprising an opening in the vicinity of their periphery. The edge (16, 26) of such opening in the plates create a sort of rod (16, 26) onto which the hooked part (63) of both removable embodiments of the osseous anchoring means (60) can be interlocked.

Figures 4 to 9 illustrate the plates (1, 2) of the prosthesis traverse equipped with their anatomic adaptation elements (11, 22) and define different embodiments of the fixation means (113, 223, 15, 25) of these anatomic adaptation elements (11, 22) on the plates (1, 2). These fixation means (113, 223, 15, 25) are reversible, this means that the anatomic adaptation elements (11, 22) can be easily attached and removed from the plates (1, 2) of the prosthesis. These fixation means (113, 223, 15, 25) allow the anatomic adaptation elements (11, 22), fixed in a moveable manner to the plates (1, 2), to be changed. The fixation means (113, 223, 15, 25) of the anatomic adaptation elements (11, 22) on the plates (1, 2) consist in fixation means (113, 223) present on the anatomic adaptation elements (11, 22) and complementary with the fixation means (15, 25) present on the plates (1, 2) of the prosthesis. The anatomic adaptation elements (11, 22) are fixed onto the plates (1, 2) via, on one hand, contact of at least a part of their lower (111) and upper (222) surface with at least a part of respectively the upper (1) and lower (2) plates and, on the other hand, contact of their fixation means (113, 223) with the complementary fixation means (15, 25) present on the plates (1, 2) of the prosthesis. For the anatomic plates (11, 22) such as those illustrated, for example, in figures 4A to 4D, the anatomic adaptation elements (11, 22) are fixed onto the plates (1, 2) thanks to the fact that the upper (10) and lower (20) surfaces of respectively the upper (1) and lower (2) plates are firmly attached to the reinforcements present on the lower (111) and upper (222) surfaces of respectively the upper (11) and lower (22) anatomic plates, via the fixation means (113, 223, 15, 25). For the anatomic crowns (11, 22) such as those illustrated, for example, in figures 5A to 5C, the anatomic adaptation elements (11, 22) are fixed onto the plates (1, 2) thanks to the fact that the bevelled parts of the upper (10) and lower (20) surfaces of respectively the upper (1) and lower (2) plates are firmly attached to the bevelled parts of the upper (111) and lower (222) surfaces of respectively the upper (11) and lower (22) anatomic plates, via the fixation means (113, 223, 15, 25). The different embodiments of the fixation means (113, 223, 15, 25) of the anatomic adaptation elements (11, 22) on the plates (1, 2) will now be described in reference to figures 4 to 9. It is obvious that these fixation means are given by way of illustration and can be replaced by any equivalent means without leaving the scope of the invention. Likewise, the invention allows the use of any whatsoever combination of the different fixation means (113, 223, 15, 25) described below.

In several embodiments, the fixation means (113, 223, 15, 25) of the anatomic adaptation elements (11, 22) on the plates (1, 2) consist in male fixation means (113, 223) present on the anatomic adaptation elements (11, 22) and co-operate with the female fixation means (15, 25) present on the plates (1, 2) of the prosthesis. The female fixation means (15, 25) present on the plates (1, 2) of the prosthesis can consist, for example, in plane surfaces (15, 25) present on the edges of the plates (1, 2) of the prosthesis or in recesses (15, 25) either made in the edges of the plates (1, 2) of the prosthesis, or in the edges of the female co-operation means (23) of the plates (1, 2) of the prosthesis.

In an embodiment illustrated in figures 4A and 4B, the fixation means (113) of the upper anatomic plate (11) consist, on the posterior edge of its lower surface (111), in nibs which are shaped and have dimensions intended to receive a section (15) of the posterior edge of the lower surface (14) of the upper plate (1). On the anterior edge of its lower surface (111), the fixation means (113) of the upper anatomic plate (11) consist in latches constituted in an axis of rotation onto which a hasp is mounted intended to swivel about this axis and receive a section (15) of the posterior edge of the lower surface (14) of the upper plate (1), as particularly visible in figures 4A and 4B. The right-hand latches in figures 4A to 4C are illustrated in the open position and the left-hand latches are in the closed position. In the embodiment illustrated in figures 4C and 4D, the fixation means (223) of the lower anatomic plate (22) consist, on the posterior edge of its lower surface (222), in nibs which are shaped and have dimensions intended to fit into an opening (25) made in the co-operation means (23) of the lower plate (2). On the anterior edge of its lower surface (222), the fixation means (223) of the upper anatomic plate (22) consist in latches constituted in an axis of rotation onto which a hasp is mounted intended to receive a recess (25) made in a part of the co-operation means (23) present on the posterior edge of the lower plate (2). The latches illustrated in figures 4A to 4D can be maintained in the closed position via securing means (55) present, for example, on the plates (1, 2) of the prosthesis. For example, as illustrated in figure 4C, a notch (55) made on the recess (25) present on a part of the co-operation means (23) of the lower plate (2) prevents the latch (223) of the lower anatomic plate (22) from swivelling.

In the embodiment in figures 5A to 5C, the anterior and posterior edges of the upper anatomic adaptation crowns (11) have fixation means (113) consisting in nibs which co-operate with a plane section (15) present on the edge of the lower surface (14) of the upper plate.

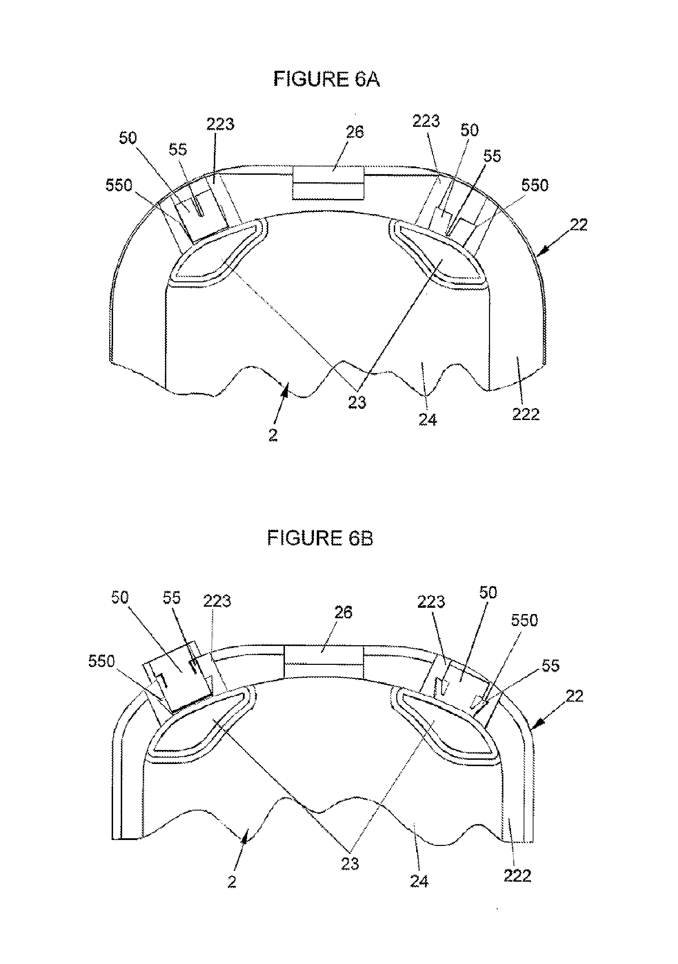

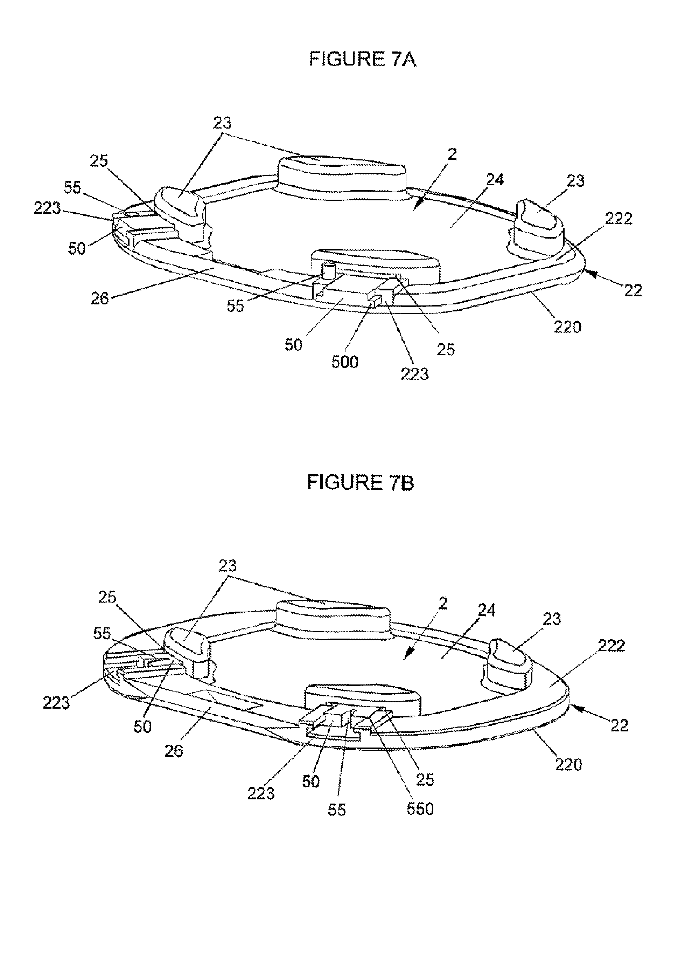

In the embodiments in figures 6A to 9D, the fixation means (113, 223, 15, 25) of the anatomic adaptation elements (11, 22) on the plates (1, 2) consist in female fixation means (113, 223) present on the anatomic adaptation elements (11, 22) and co-operating with male intermediary means (50) which can also co-operate with the female fixation means (15, 25) present on the plates (1, 2) of the prosthesis. The anatomic adaptation elements (11, 22) are fixed onto the plates (1, 2) via, on one hand, contact of at least a part of their upper (111) and lower (222) surface with at least a part of respectively the upper (1) and lower (2) plates and, on the other hand, contact of the male intermediary means (50) with the female fixation means (113, 223) present on the anatomic adaptation elements (11, 22) and with the female fixation means (15, 25) present on the plates (1, 2) of the prosthesis. The male intermediary means (50) consist in a sliding plate (50) in the female fixation means (113, 223) present on the anatomic adaptation elements (11, 22) to co-operate with the female fixation means (15, 25) present on the plates (1, 2) of the prosthesis. The plate (50) is substantially parallelepiped in shape and can comprise, on its side edges, fins (500), particularly visible, for example, in figure 7A. These fins (500) of the male intermediary means (50) are of complementary shape with the female fixation means (113, 223) of the anatomic adaptation elements (11, 22) and with the female fixation means (15, 25) of the plates (1, 2) of the prosthesis, which have side runners in which these fins (500) slide. This complementary shape of the fins (500) of the plate (50) and the runners of the female fixation means (113, 223) of the anatomic adaptation elements (11, 22) as well as the female fixation means (15, 25) of the plates (1, 2) prevent the plate (50) from leaving these female fixation means (113, 223, 15, 25) prior to being blocked via the securing means (55).

The male intermediary means (50) have securing means (55) blocking the male intermediary means (50) in the position where they co-operate with both the female fixation means (113, 223) of the anatomic adaptation elements (11, 22) and with the female fixation means (15, 25) present on the plates (1, 2) of the prosthesis. These securing means (55) which consist, for example, in at least a formal irregularity (55) present on at least one side of this plate (50) and intended to co-operate with at least an opening (550) made in the female fixation means (113, 223) of the anatomic adaptation elements (11, 22) and/or in the female fixation means (15, 25) of the plates (1, 2). The opening (550) can be of a complementary shape of the male intermediary means (50) or of its securing means, as illustrated in figures 6A and 6B.

In the embodiment illustrated in figure 6A, the plate constituting the male intermediary means (50) widens out towards its posterior end and the formal irregularity constituting the securing means (55) consists in a slot on the posterior half of the plate (50). This slot (55) compresses the posterior end of the plate (50) when it is introduced into the female fixation means (113, 223) of the upper and/or lower anatomic adaptation elements (11, 22), as illustrated for the left-hand plate in figure 6A. When the plate (50) reaches its end of stroke in the runner created by the female means (113, 223) of the anatomic adaptation elements (11, 22) and the means (15, 25) of the plates (1, 2), that meaning when the it co-operates with these two female means at the same time, openings (550) made, for example, in the female means (113, 223) of the anatomic adaptation elements (11, 22) separate the plate (50) from its hold, as illustrated for the right-hand plate in figure 6A. Figure 7B illustrates a perspective view of this embodiment of the fixation means in which the plate (50) is intended to be held in the female manes (25) made in the co-operation means (23) of the lower plate (2). This figure also notably shows the fact that the reinforcement present, for example, on the lower anatomic plate (22) can be deeper than the thickness of the lower plate (2). Depending on the size of the co-operation means (23, 33) of the lower plate (2) of the core (3), the edges of this reinforcement will provide a periphery abutment possibly limiting the displacement of the core (3) in relation to the lower plate (2). In the embodiment illustrated in figure 6B, the formal irregularities constituting the securing means (55) of the male intermediary means (50) consist in hasps present on the side edges of the plate (50). As illustrated for the left-hand plate (50) in figure 6B, these hasps (55) are compressed when the hasp is introduced into the runners of the female means (113, 223). When the plate is pushed as far as the blocking position, the hasps (55) naturally open out in the openings (550) provided for on the side edges of the female means (113, 223) of the upper and/or lower anatomic adaptation elements (11, 22), as illustrated for the right-hand plate (50) in figure 6B.

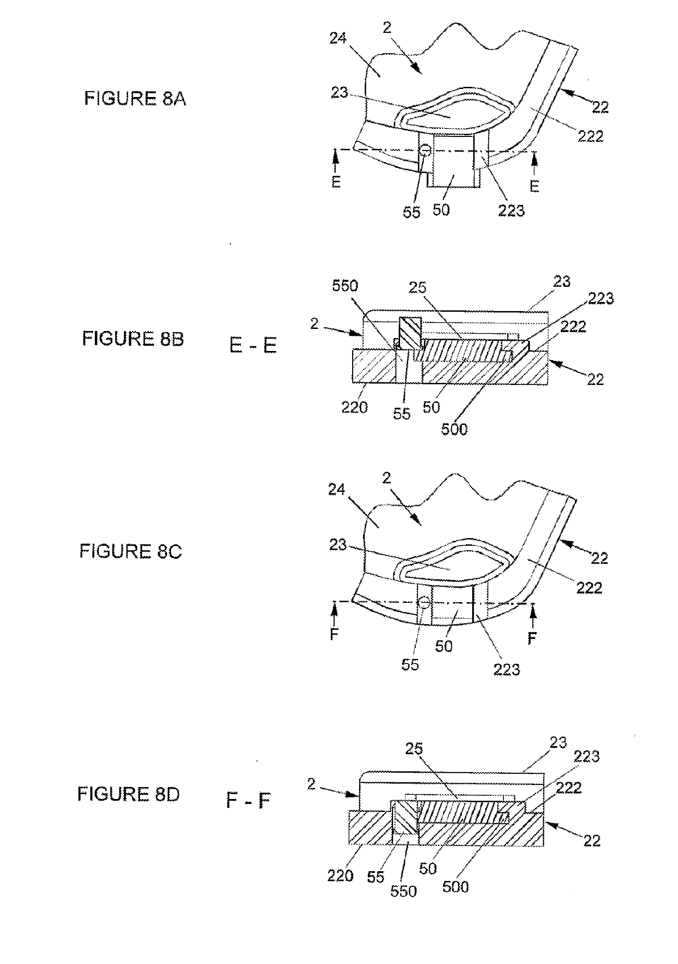

Figures 7A and 8A to 8D illustrate another alternative embodiment of the male intermediary means (50). In this embodiment, the formal irregularities of the plate (50) constituting the securing means (55) of the plate (50) consist in a bore in the male intermediary means (50), prolonged by a bore (550) in the female fixation means (113, 223) of the anatomic adaptation elements (11, 22), as particularly visible in figure 8B. The bore (550) is intended to receive a securing pin (55) blocking the male intermediary means (50) in the position where they co-operate with the female fixation means (15, 25) present on the plates (1, 2) of the prosthesis, as illustrated in figure 8C.

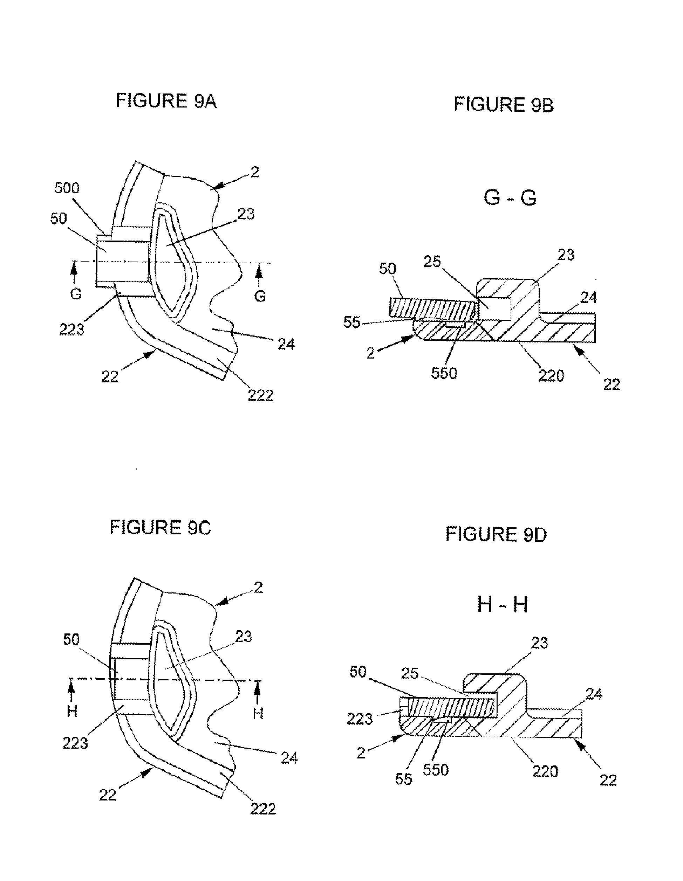

Another alternative of the securing means (55) of the male intermediary means (50) is illustrated in figures 9A to 9D. In this alternative, the formal irregularities constituting the securing means (55) of the plate (50) consist in a notch (55) present on the lower surface of the plate and intended to co-operate with an opening (550) made in the female fixation means (113, 223) of the anatomic adaptation elements by resisting against the removal of the plate (50), once driven as far as the female fixation means (15, 25) of the plates (1, 2), as illustrated in figure 9D.

It must be evident for specialists that the invention allows embodiments in numerous other specific forms without departing from the scope of application of the invention as claimed. As a consequence, the embodiments must be considered by way of illustration, but can be modified within the scope defined by the range of the attached claims, and the invention does not have to be limited to the details given above.

## Claims

1. Osseous anchor (60) for anchoring an intervertebral implant in osseous tissue of a vertebra, wherein the anchor includes a plate (61) having a far end bearing a part (63) curved to fold over itself which is able to be interlocked onto means for clasping the osseous anchor (60) on said implant, so as to retain said implant against said osseous tissue, while allowing removal of the osseous anchor (60) from the implant, **characterised in that** the plate has an overall elongated rectangular shape of substantially constant breadth and thickness.

2. Osseous anchor (60) according to claim 1, **characterized in that** said curved part (63) is able to be interlocked onto said means for clasping on said implant formed by an edge (16, 26) of an opening made in the vicinity of the periphery of said implant.

3. Osseous anchor (60) according to claim 2, **characterized in that** said plate (61) is able to penetrate the osseous tissue through said opening of said implant.

4. Osseous anchor (60) according to any of claims 1 to 3, **characterized in that** said curved part (63) is able to be interlocked onto said means for clasping on said implant formed by a rod at an edge (16, 26) of an opening made in the vicinity of the periphery of said implant, such that the osseous anchor (60) is movable and allows adjusting the angle of said osseous anchor (60) relative to the implant, by rotation around said rod.

5. Osseous anchor (60) according to any of claims 1 to 3, **characterized in that** said curved part (63) prolong with a second plate (61).

6. Osseous anchor (60) according to claim 5, **characterized in that** the second plate is smaller than the first plate.

7. Osseous anchor (60) according to any one of claims 1 to 6, **characterized in that** the first and/or second plate(s) (61) is/are equipped with notches (62) oriented to resist against the removal of the osseous anchor (60) from the vertebra.

8. Intervertebral disc prosthesis and osseous anchor (60) for anchoring said intervertebral disc prosthesis, **characterized in that** said osseous anchor is an osseous anchor according to claim 1 to 4 and **in that** the breadth of the plate (61) corresponds to the breadth of an opening made in the vicinity of the periphery of the intervertebral disc prosthesis and having an edge (16, 26) forming said means for clasping the osseous anchor (60) on said implant.

9. Intervertebral disc prosthesis and osseous anchor (60) for anchoring said intervertebral disc prosthesis **characterized in that** said osseous anchor is an osseous anchor according to any of claims 5 or 6 and **in that** the breadth of at least one of the plates (61) corresponds to the breadth of an opening made in the vicinity of the periphery of the intervertebral disc prosthesis and having an edge (16, 26) forming said means for clasping the osseous anchor (60) on said implant.

10. Intervertebral disc prosthesis and osseous anchor (60) according to claim 7 or 8, **characterized in that** said clasping means of said implant are intended to be in contact with the osseous tissue, such that said plate (61) of the anchor penetrates into the osseous tissue up to its far end.

11. Intervertebral disc prosthesis and osseous anchor (60) according to any of claims 7 to 9, **characterized in that** said implant is an anatomic adaptation element (11, 22) extending the overall diameter and/or the overall height of an intervertebral disc prosthesis and having clasping means onto which said curved part (63) is able to be interlocked.

## Patentansprüche

1. Knochenanker (60) zum Verankern eines Intervertebralimplantats in Knochengewebe eines Wirbels, wobei der Anker eine Platte (61) mit einem entfernten Ende aufweist, das ein Teil (63) trägt, das, um umzuklappen, gekrümmt ist, um auf sich selbst zu klappen, das an Mitteln zum Greifen des Knochenankers (60) an dem Implantat verriegelt werden kann, um das Implantat an dem Knochengewebe zu halten, während die Entnahme des Knochenankers (60) von dem Implantat ermöglicht wird, **dadurch gekennzeichnet, dass** die Platte eine langgestreckte rechteckige Gesamtform mit im Wesentlichen konstanter Breite und Dicke aufweist.

2. Knochenanker (60) nach Anspruch 1, **dadurch gekennzeichnet, dass** dieser gekrümmte Teil (63) an diese Mittel zum Greifen dieses Implantats, die von einem Rand (16, 26) einer in der Umgebung des Umfangs dieses Implantats gebildeten Öffnung gebildet werden, verriegelt werden kann.

3. Knochenanker (60) nach Anspruch 2, **dadurch gekennzeichnet, dass** diese Platte (61) durch diese Öffnung dieses Implantats in das Knochengewebe eindringen kann.

4. Knochenanker (60) nach einem der Ansprüche 1 bis 3, **dadurch gekennzeichnet, dass** dieser gekrümmte Teil (63) an diesen von einem Stab an einem Rand (16, 21) einer in der Umgebung des Umfangs des Implantats erzeugten Öffnung gebildeten Mitteln zum Greifen an dem Implantat verriegelt werden kann, so dass der Knochenanker (60) beweglich ist und das Verstellen des Winkels des Knochenankers (60) relativ zu dem Implantat durch Drehung um den Stab ermöglicht.

5. Knochenanker (60) nach einem der Ansprüche 1 bis 3, **dadurch gekennzeichnet, dass** dieser gekrümmte Teil (63) mit einer zweiten Platte (61) verlängert ist.

6. Knochenanker (60) nach Anspruch 5, **dadurch gekennzeichnet, dass** die zweite Platte kleiner ist als die erste Platte.

7. Knochenanker (60) nach einem der Ansprüche 1 bis 6, **dadurch gekennzeichnet, dass** die erste und/oder die zweite Platte (61) mit Kerben (62) ausgestattet ist/sind, die orientiert sind, um dem Entnehmen des Knochenankers (60) aus dem Wirbel zu widerstehen.

8. Bandscheibenprothese und Knochenanker (60) zum Verankern dieser Bandscheibenprothese, **dadurch gekennzeichnet, dass** es sich bei dem Knochenanker um einen Knochenanker nach Anspruch 1 bis 4 handelt und dass die Breite der Platte (61) der Breite einer Öffnung entspricht, die in der Umgebung des Umfangs der Bandscheibenprothese erzeugt ist und einen Rand (16, 26) aufweist, der diese Mittel zum Greifen des Knochenankers (60) an dem Implantat bildet.

9. Bandscheibenprothese und Knochenanker (60) zum Verankern dieser Bandscheibenprothese, **dadurch gekennzeichnet, dass** es sich bei diesem Knochenanker um einen Knochenanker nach einem der Ansprüche 5 bis 6 handelt und dass die Breite mindestens einer der Platten (61) der Breite einer Öffnung entspricht, die in der Umgebung des Umfangs der Bandscheibenprothese erzeugt ist und einen Rand (16, 26) aufweist, der die Mittel zum Greifen des Knochenankers (60) an diesem Implantat bildet.

10. Bandscheibenprothese und Knochenanker (60) nach Anspruch 7 oder 8, **dadurch gekennzeichnet, dass** die Greifmittel dieses Implantats dazu bestimmt sind, derart mit dem Knochengewebe in Kontakt zu sein, dass die Platte (61) des Ankers bis zu ihrem entfernten Ende in das Knochengewebe eindringt.

11. Bandscheibenprothese und Knochenanker (60) nach einem der Ansprüche 7 bis 9, **dadurch gekennzeichnet, dass** es sich bei dem Implantat um ein anatomisches Anpassungselement (11, 22) handelt, das den Gesamtdurchmesser und/oder die Gesamthöhe einer Bandscheibenprothese ausdehnt und Greifmittel aufweist, an denen der gekrümmte Teil (63) verriegelt werden kann.

## Revendications

1. Ancrage osseux (60) pour ancrer un implant intervertébral dans le tissu osseux d'une vertèbre, dans lequel l'ancrage comprend un plateau (61) ayant une extrémité éloignée supportant une partie (63) incurvée pour se replier sur elle-même qui peut être enclenchée sur des moyens pour serrer l'ancrage osseux (60) sur ledit implant, afin de retenir ledit implant contre ledit tissu osseux, tout en permettant le retrait de l'ancrage osseux (60) de l'implant, **caractérisé en ce que** le plateau a une forme rectangulaire allongée générale de largeur et d'épaisseur sensiblement constantes.

2. Ancrage osseux (60) selon la revendication 1, **caractérisé en ce que** ladite partie incurvée (63) peut être enclenchée sur lesdits moyens pour serrer ledit implant formé par un bord (16, 26) d'une ouverture réalisée à proximité de la périphérie dudit implant.

3. Ancrage osseux (60) selon la revendication 2, **caractérisé en ce que** ledit plateau (61) peut pénétrer dans le tissu osseux par ladite ouverture dudit implant.

4. Ancrage osseux (60) selon l'une quelconque des revendications 1 à 3, **caractérisé en ce que** ladite partie incurvée (63) peut être enclenchée sur lesdits moyens pour serrer ledit implant formé par une tige au niveau d'un bord (16, 26) d'une ouverture réalisée à proximité de la périphérie dudit implant, de sorte que l'ancrage osseux (60) est mobile et permet d'ajuster l'angle dudit ancrage osseux (60) par rapport à l'implant, par rotation autour de ladite tige.

5. Ancrage osseux (60) selon l'une quelconque des revendications 1 à 3, **caractérisé en ce que** ladite partie incurvée (63) se prolonge avec un second plateau (61).

6. Ancrage osseux (60) selon la revendication 5, **caractérisé en ce que** le second plateau est plus petit que le premier plateau.

7. Ancrage osseux (60) selon l'une quelconque des revendications 1 à 6, **caractérisé en ce que** le premier et/ou le second plateau (61) est/sont équipé(s) avec des encoches (62) orientées afin de résister au retrait de l'ancrage osseux (60) de la vertèbre.

8. Prothèse de disque intervertébral et ancrage osseux (60) pour ancrer ladite prothèse de disque intervertébral, **caractérisés en ce que** ledit ancrage osseux est un ancrage osseux selon les revendications 1 à 4, et **en ce que** la largeur du plateau (61) correspond à la largeur d'une ouverture réalisée à proximité de la périphérie de la prothèse de disque intervertébral et ayant un bord (16, 26) formant lesdits moyens pour serrer l'ancrage osseux (60) sur ledit implant.

9. Prothèse de disque intervertébral et ancrage osseux (60) pour ancrer ladite prothèse de disque intervertébral, **caractérisés en ce que** ledit ancrage osseux est un ancrage osseux selon l'une quelconque des revendications 5 ou 6, et **en ce que** la largeur d'au moins l'un des plateaux (61) correspond à la largeur d'une ouverture réalisée à proximité de la périphérie de la prothèse de disque intervertébral et ayant un bord (16, 26) formant lesdits moyens pour serrer l'ancrage osseux (60) sur ledit implant.

10. Prothèse de disque intervertébral et ancrage osseux (60) selon la revendication 7 ou 8, **caractérisés en ce que** lesdits moyens de serrage dudit implant sont prévus pour être en contact avec le tissu osseux, de sorte que ledit plateau (61) de l'ancrage pénètre dans le tissu osseux jusqu'à son extrémité éloignée.

11. Prothèse de disque intervertébral et ancrage osseux (60) selon l'une quelconque des revendications 7 à 9, **caractérisés en ce que** ledit implant est un élément d'adaptation anatomique (11, 22) étendant le diamètre total et/ou la hauteur totale d'une prothèse de disque intervertébral et ayant des moyens de serrage sur lesquels ladite partie incurvée (63) peut être enclenchée.
